# EUROPEAN PATENT APPLICATION

(11) **EP 4 696 264 A1**
(43) Date of publication of application: **18.02.2026**
(21) Application number: 24194192.1
(22) Date of filing: 12.08.2024
(51) Int. Cl.: A61B 18/18

(54) **A MICROWAVE ABLATION PROBE**

(71) Applicant: Endowave Ltd., D02 DK18 Dublin 2 (IE)
(72) Inventor: RUVIO, Giuseppe, Dublin 2, D02 DK18 (IE); EATON-EVANS, Jimmy, Dublin 2, D02 DK18 (IE); WALSH, Eoin J., Dublin 2, D02 DK18 (IE); HOGAN, Simon, Dublin 2, D02 DK18 (IE); COMER, James, Dublin 2, D02 DK18 (IE); FARINA, Laura, Dublin 2, D02 DK18 (IE); WALSH, Paul, Dublin 2, D02 DK18 (IE)
(74) Representative: Barker Brettell LLP

(57) **Abstract**

A microwave ablation probe (100), comprising: an applicator (106) having a radiating section (106a) arranged to emit electromagnetic energy to cause heating in tissue; a feed cable (108) having a distal end and a proximal end and being arranged to supply electromagnetic energy to the applicator (106), the applicator (106) being coupled to the distal end of the feed cable (108); a flexible tubular member (110) comprising a shaft portion (111) and a tip portion (112), the tip portion (112) located at a distal end of the tubular member (110) and being adapted for insertion into tissue, wherein the applicator (106) and at least part of the feed cable (108) are located within the tubular member (110); and a restriction means (114) arranged to restrict the separation between the applicator (106) and the tip portion (112) of the tubular member (110). The restriction means (114) comprises a flexible tethering member (116) extending between the applicator (106) and the tip portion (112), the tethering member (116) being arranged to restrict at least proximal movement of the applicator (106) relative to the tip portion (112) of the tubular member (110). Also disclosed is a method (1000) of manufacturing the ablation probe (100).

## Description

This application relates to a microwave ablation probe and a method of manufacturing a microwave ablation probe. The ablation probe can be delivered using a minimally invasive procedure to the target. In particular, this application relates to a flexible microwave ablation catheter probe comprising an applicator arranged to emit electromagnetic energy and a restriction means arranged to restrict relative movement of the applicator within the ablation probe. The application further relates to a method of manufacturing the ablation probe.

Thermal ablation can be used to destroy tissue growths within the body which can be malignant. Current ablation systems use applicators that deliver Radio Frequency (RF) energy or more specifically microwave energy to the tissue surrounding an applicator tip. This causes localised heating and destruction of the malignant cells.

Known ablation probes comprise an active tip (or applicator) generally made of ceramic material which is coupled to a coaxial feed cable arranged to supply electromagnetic energy to the active tip. The applicator and feed cable are housed within a catheter tube, which can then be inserted through a delivery device such as an endoscope to reach an ablation site within the anatomy of a patient.

The design of such an ablation probe is challenging because of the tortuous path the probe may need to follow to reach the ablation site. This may be particularly so where the ablation site is inside the lungs of the patient. The ablation probe must be made sufficiently small and flexible so that it can inserted along the working channel of a delivery device. Moreover, different components can change shape in different ways and move relative to each other when the ablation probe is bent. Despite this, it is also important that the applicator does not become detuned from the designated operating frequency and that the ablation zone produced can be accurately positioned in the target to ensure the ablation zone does not unintentionally damage critical anatomical structures which may be close by.

The present application provides an ablation probe having an applicator aimed at overcoming one or more of these challenges.

A first aspect of the present application provides a microwave ablation probe, comprising any one or more of the following features:
- an applicator having a radiating section arranged to emit electromagnetic energy to cause heating in tissue;
- a feed cable having a distal end and a proximal end and being arranged to supply electromagnetic energy to the applicator, the applicator being coupled to the distal end of the feed cable;
- a flexible tubular member, comprising a shaft portion and a tip portion, the tip portion located at a distal end of the tubular member and being adapted for insertion into tissue, wherein the applicator and at least part of the feed cable are located within the tubular member; and
- a restriction means arranged to restrict the separation between the applicator and the tip portion of the tubular member,
wherein the restriction means comprises a flexible tethering member extending between the applicator and the tip portion, the tethering member being arranged to restrict at least proximal movement of the applicator relative to the tip portion of the tubular member.

The restriction means is arranged to restrict the range of possible relative movement between the applicator and the tip of the tubular member during use of the ablation probe. This may control the movement between the applicator and the tip when the ablation probe is inserted along a tortuous path through the patient's anatomy. The restriction means may help to locate the tip of the ablation probe relative to the applicator during use and means that the ablation zone generated by the applicator can be positioned accurately while avoiding the tip damaging critical anatomical structures. The control of the relative position of components within the tubular member (e.g. the applicator) is further advantageous because it means that navigation through the tortuous path of the natural lumen doesn't change the energy delivery performance of the applicator.

An ablation procedure may be carried out using the ablation probe of the present application under image-guidance (such as X-ray image guidance e.g. fluoroscopy, Computed Tomography or Cone-Beam Computed Tomography; or ultrasound image guidance). To run the procedure the clinician needs to align the expected centre of the ablation zone with the centre of the target lesion. While the clinician does this, she/he needs to make sure that the tip of the ablation probe is at safe distance from critical structures (such as pleura in the lung or critical vessels). The centre of the ablation zone can be estimated from the location of the applicator and/or other markers visible using the image guidance. By fixing the applicator relative to the tip of the ablation probe to within an acceptable tolerance the ablation zone can be positioned and the relative location of the tip known to avoid damaging critical tissue. If the tip could move freely relative to the applicator during insertion of the ablation probe to reach the ablation site, the clinician would no longer know the spatial relationship between them. If they were then to correctly align the ablation probe by monitoring the position of the applicator, the tip may inadvertently come into contact with critical anatomical structures because the clinician would not know to a high enough accuracy where it is located.

The tubular member (including the tip) may be flexible to navigate through the tortuous path of natural anatomical lumens (e.g. airways) to reach the ablation zone. The structure(s) of the restriction means may be arranged not to inhibit this flexibility, while still controlling the position of the components within the tubular member. The restriction means may therefore provide a flexible, indirect, coupling between the applicator and the tip portion of the tubular member.

At least a distal region of the feed cable may otherwise be free to move relative to the part of the tubular member in which it is contained. In other words, it may not be restricted from moving (e.g. proximally or distally) within the tubular member apart from by the restriction means or the interior walls of the tubular member.

The restriction means may be referred to as an applicator movement restricting mechanism or anchoring mechanism as it restricts or anchors the position of the applicator within the ablation probe relative to the tip.

The tethering member may limit proximal migration of the applicator (in particular the radiating section) relative to the tip portion so that the spatial relationship between them is known. If the applicator were instead rigidly coupled directly to the tip the overall flexibility of the ablation probe would be reduced as the combined applicator and tip would form an extended length of relatively rigid material.

The tethering member may comprise a flexible filament extending between the applicator and the tip portion of the tubular member. By using a flexible filament, the overall flexibility of the ablation probe can be maintained so as not to reduce the amount which it can be bent to reach the ablation site.

The tethering member may be arranged to preserve its mechanical properties at high temperature. The tethering member may comprise (i.e. may be made from) an electrically insulating material. This may help to avoid electromagnetic interference with the applicator and over-heating during the ablation procedure.

The tethering member may be formed from a polymeric material. Preferably, the tethering member may comprise fluorinated ethylene propylene (FEP). This may provide suitable flexibility and ease of manufacture.

The tethering member may be formed from the same material as the tip portion of the tubular member. The tethering member may be embedded within the material of the tip portion. This may provide a strong and secure bond between them.

The tip portion and tethering member may be reflow-bonded together. This may provide a secure bond and may be particularly advantageous where they comprise the same material. The use of reflow bonding may avoid the need to use adhesives or more complex mechanical retention features.

The tubular member may comprise a tip component coupled to the shaft portion of the tubular member. The tip portion may therefore be formed from a separate component attached to body of the tubular member.

The shaft portion, tip component and tethering member may all be formed from (i.e. they may all comprise) the same material. This may allow them to be more effectively bonded together.

The shaft portion, tip component and tethering member may be reflow-bonded together.

The restriction means may be further arranged to restrict distal movement of the radiating section of the applicator relative to the tip portion of the tubular member. This may prevent the radiating section from getting too close to the tip during use. In particular, this may otherwise occur when the ablation probe is bent to reach an ablation site. By restricting how close the applicator can get to the tip portion the risk of the applicator detuning may be reduced and the correct dose of radiation is provided.

The restriction means may comprise an abutment portion of the applicator. The abutment portion may be arranged to space apart the radiating section of the applicator from the most distal point of the applicator. The abutment portion may contact the tip portion of the tubular member (e.g. during use) and form a minimum spacing between the radiating section and the tip portion. The abutment portion may be advantageous in providing pushability of the ablation probe i.e. it allows the ablation probe to be effectively pushed along a working channel from its proximal end.

The abutment portion may comprise an anchor point for the tethering member. This may provide a suitable location to attach the tethering member and allows the abutment portion to perform two functions. This may reduce the overall size of the ablation probe.

The anchor point may comprise a hole in the abutment portion arranged to couple with the tethering member. This may provide a secure and compact coupling between the applicator and the tip portion.

The ablation probe may comprise one or more markers comprising a marker material that is visible using an associated imaging system (e.g. an image guidance system) during use of the ablation probe. The marker material may be a material which is radiopaque and/or echogenic. The radiopaque material may comprise barium sulphate.

At least one of the one or more markers may be located at a known fixed position relative to the centre of the ablation zone produced by the applicator.

At least one of the one or more markers may be located at a known fixed position relative to tip portion.

By providing a marker at a fixed position relative to the tip and/or the applicator the relative position of both components can be tracked during use because of the relationship set between them by the restriction means. For example, if the location of the applicator can be determined during use, the ablation zone can be positioned accurately. The relative location of the tip will also be known because its movement relative to the applicator is restricted by the restriction means. This allows damage to critical anatomical tissue to be avoided. A similar effect can be achieved by the tip comprising a marker and the associated position of the applicator set by the restriction means. In some cases, both the applicator and tip may comprise markers.

This may allow the user to conveniently locate part of the ablation probe relative to the patient's anatomy during use. The markers may help the accurate delivery of the ablation probe by providing visible feedback of the location of the ablation zone and /or distance from the distal end of a working channel of a delivery device through which the ablation probe is inserted in relation to the tip of the tubular member.

The inventors have identified that the clinician must align the centre of the ablation zone with the centre of the tumour being ablated and ensure the tip of the tubular member is placed at a safe distance from critical structures (e.g. the pleura). To achieve this, the reference marking the centre of the ablation zone is at a fixed distance from the tip portion because of the restriction means and clearly identifiable under image guidance.

At least one of the one or more markers may be located at a position corresponding to a point where the radiation emitted by the applicator is below a threshold safe level. At least one of the one or more markers (and hence the distal end of the working channel) may be located about 1 cm from the margin of the ablation zone in its largest operating setting. This may mark the position on the ablation probe far enough away from the centre of the ablation zone where the radiation has fallen to a safe level. For example, this may be a level at which the working channel through which the ablation probe is delivered may be safe from damage by radiation emitted by the ablation probe. This may help ensure that the ablation probe is extended sufficiently from the distal end of the working channel before the ablation is activated.

The one or more markers may be formed by a part or parts of the ablation probe which is made from an (or the) indicator material.

The tip portion may comprise an indicator material and may act as one of the one or more markers. The tip portion may comprise a polymeric material doped with an indicator material (e.g. a radiopaque and/or echogenic material). This may allow the tip portion itself to act as a marker without an additional marker being needed.

Additionally, or alternatively, the applicator may comprise a dielectric resonator comprising an indicator material (e.g. a radiopaque material and/or echogenic material) and may act as one of the one or more markers. The dielectric resonator may comprise a radiopaque ceramic. This may allow the applicator itself to act as a marker without an additional marker being needed.

The one or more markers may include one or more marker components secured to an associated component of the ablation probe.

The one or more marker components may each comprise a band extending around a respective component of the ablation e.g. around the feed cable.

The one or more marker components may each comprise a metallic band.

The ablation probe may further comprise a coolant circuit extending along the length of the tubular member and feed cable. The coolant circuit may extend through the restriction means to allow passage of coolant around the distal end of the applicator. The coolant circuit may carry a coolant from the proximal end of the ablation probe, along its length to the applicator and back again to provide cooling. By forming the restriction means by a thin flexible component (e.g. a filament) coolant can flow through the restriction means without significant impedance to the flow. The presence of water all around the antenna helps to stabilize its behaviour. If the applicator was integral with the catheter tip rather than being coupled as described in the present application, the coolant would not reach the distal end of the antenna. This would reduce the performance of the ablation probe.

The feed cable may comprise a coaxial cable comprising an inner conductor, an outer conductor and a dielectric therebetween.

The applicator may comprise an antenna section formed integrally with the inner conductor of the feed cable. The antenna section may be wrapped around an outer surface of the applicator and may be mechanically coupled to the applicator by one or more retention features on the outer surface of the applicator. The one or more retention features may retain the antenna section without the need for adhesive or other fixing. The one or more retention features may be provided on the dielectric resonator of the applicator. The mechanical coupling between the applicator and the feed cable may balance or counteract the force applied to applicator by the tethering member.

The one or more retention features may comprise a groove or cut-out in the outer surface of the dielectric resonator.

The applicator may comprise a helical antenna. The helical antenna may comprise: a straight section arranged to extend through an axial hole in the dielectric resonator of the applicator; a radially extending section extending through a radial hole in the dielectric resonator and a helical section extending around an outer surface of the dielectric resonator.

The straight section, radially extending section and helical section may be integrally formed with the inner conductor of the feed cable.

The dielectric resonator may comprise a channel configured to receive the helical section of the antenna. The channel may act as a retention feature as defined above. The channel may extend along a helical path around a longitudinal axis of the dielectric resonator. The channel may maintain the shape of the helical channel.

Engagement of the antenna sections with the radially extending hole and helical channel may provide a coupling between the part of inner conductor forming the antenna and the dielectric resonator.

The dielectric resonator may comprise a plurality of fins formed on its outer surface. The plurality of fins may be configured to abut an inner surface of the tubular member such that the dielectric resonator and tubular member are aligned concentrically.

The dielectric resonator may comprise an aperture formed in a proximal end of the dielectric resonator. The aperture may be configured to receive an exposed portion of the dielectric and the corresponding part of the inner conductor of the feed cable.

The applicator may comprise a dipole antenna. The dipole antenna may comprise a first antenna section and a second antenna section.

The first antenna section may comprise the helical antenna.

The second antenna section may be located proximally along the length of the ablation probe relative to the first antenna section.

The second antenna section may comprise a plurality of antenna elements which are integral with the outer conductor of the feed cable. The antenna elements may be formed from part of the outer conductor which is bent-back such that it extends back proximally along the length of the feed cable.

The outer conductor may be a braided conductor. The antenna elements may comprise an unbraided part of the outer conductor.

The second antenna section may comprise one of more retaining bands arranged to secure the antenna elements. The one or more retaining bands may act as the (or some) of the one or more markers.

The applicator may comprise a flexible hinge region. A proximal end of the dielectric resonator may be spaced apart from the distal end of the outer conductor of the feed cable along the length of the feed cable to form the hinge region between them.

According to a second aspect, there is provided a microwave ablation probe, comprising any one or more of the following features:
- an applicator having a radiating section arranged to emit electromagnetic energy to cause heating in tissue;
- a feed cable having a distal end and a proximal end and being arranged to supply electromagnetic energy to the applicator, the applicator being coupled to the distal end of the feed cable;
- a flexible tubular member, comprising a shaft portion and a tip portion, the tip portion located at a distal end of the tubular member and being adapted for insertion into tissue.

The ablation probe may comprise a restriction means arranged to restrict the separation between the electrical assembly and the tip of the tubular member.

At least a distal region of the feed cable may otherwise be free to move relative to the part of the tubular member in which it is contained (e.g. it is not restricted from moving proximally or distally within the tubular member apart from by the restriction means).

The restriction means may comprise the tethering member and/or the abutment portion defined above in connection with the first aspect.

Any of the features defined above in connection with the first aspect may also apply to the second aspect.

According to a third aspect, there is provided a method of manufacturing a microwave ablation probe, the microwave ablation probe comprising:
- an applicator having a radiating section arranged to emit electromagnetic energy to cause heating in tissue;
- a feed cable having a distal end and a proximal end and being arranged to supply electromagnetic energy to the applicator, the applicator being coupled to the distal end of the feed cable;
- a flexible tubular member comprising a shaft portion and a tip portion, the tip portion located at a distal end of the tubular member and being adapted for insertion into tissue, wherein the applicator and at least part of the feed cable are located within the tubular member; and
- a restriction means arranged to restrict the separation between the applicator and the tip portion of the tubular member, wherein the restriction means comprises a tethering member extending between the applicator and the tip portion, the tethering member being arranged to restrict proximal movement of the applicator relative to the tip portion of the tubular member.

The method may comprise one or both of:
a) bonding the tethering member and the tip portion by reflowing the material of the tethering member and the tip portion; and
b) making the tip portion visible under image guidance by reflowing the material of the tip portion with a marker material.

The method of the third aspect may involve manufacturing the ablation probe of the first aspect or the second aspect.

Bonding the tethering member and tip portion and making the tip portion visible under image guidance may be carried out in a single operation.

The tethering member and tip portion may comprise the same material. They may, for example, both comprise a polymeric material (e.g. FEP).

The marker material may be a radiopaque and/or echogenic material.

The marker material may comprise the same material as the tip portion. The marker material may comprise the same material as the tip portion, that material being doped with a maker material (e.g. barium sulphate).

The tubular member may comprise a tip component forming the tip portion. The method of the third aspect may comprise reflow bonding the tip component and the shaft portion.

Bonding the tip component and the shaft portion may be carried out as part of the step of bonding the tip portion and tethering member and/or making the tip portion visible under image guidance. For example, they may be performed as a single reflow process.

Any features described above in connection with one aspect or statement may be used, unless where mutually exclusive, in combination with any other aspect or statement.

As used herein, a range "from value X to value Y" or "between value X and value Y", or the like, denotes an inclusive range; including the bounding values of X and Y. Angles are given in degrees unless otherwise stated.

Embodiments of the invention will now be described, by way of example only, with reference to the accompanying drawings, in which:
**Figure 1** shows a schematic view of an ablation probe comprising an applicator restriction means;
**Figures 2a and 2b** show cross-sectional views of the antenna region of an ablation probe without an applicator restriction means;
**Figure 3** shows the ablation probe of Figure 1 positioned within the anatomy of a patient;
**Figures 4, 5** **and** **6** show a close-up view of the ablation probe of Figure 1 showing the region around the applicator;
**Figure 7** shows a close-up view of the region around the applicator of an ablation probe having a coolant circuit;
**Figure 8** shows a schematic view of an ablation probe comprising an applicator restriction means and a helical antenna;
**Figures 9a to 9d** show views of a dielectric resonator provided in the ablation probe of Figure 8;
**Figure 10** shows a cut away side view of an ablation probe comprising a dipole antenna; and
**Figure 11** shows a method of manufacturing an ablation probe.

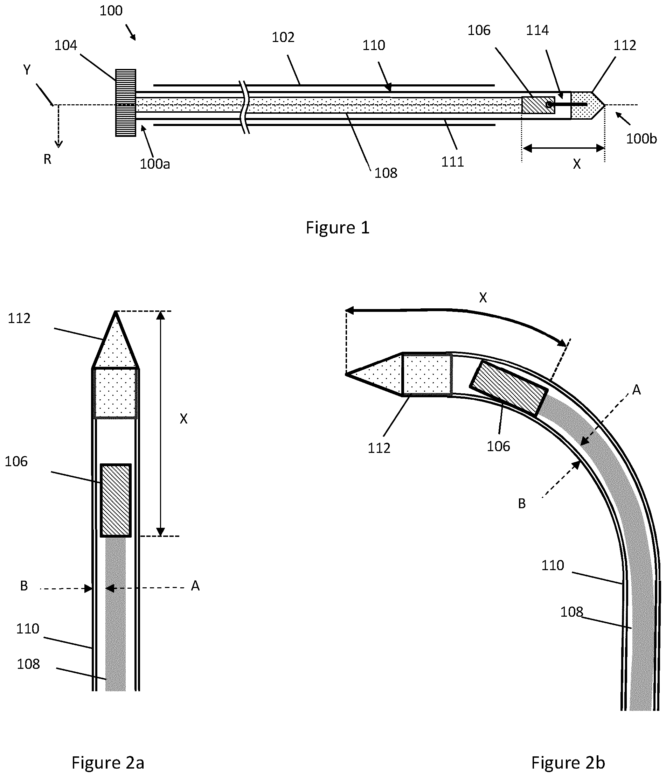

An ablation probe 100 according to an embodiment is shown schematically in Figure 1. The ablation probe 100 of the present disclosure may be suitable for insertion into the body to reach a desired treatment site, such as a malignant tissue growth. To reach a desired treatment site, the ablation probe 100 is suitable for insertion through the working channel 102 of an internal anatomy access device. By internal anatomy access device we mean any device which may be placed within the anatomy of a patient, the device having a working channel for insertion of instruments to a desired location within the body. The internal anatomy device may be an intraluminal delivery device arranged to be delivered along an anatomical lumen of the patient (e.g. the trachea and the pathways of the bronchi in the lungs or the oesophagus). The ablation probe 100 may, for example, be used endoscopically or using a bronchoscopy platform including an ENB (electromagnetic navigation bronchoscopy) or a robotic system to reach a variety of disease locations within the body. The ablation probe may therefore have an overall flexibility such that it can be inserted through the working channel of an endoscope or similar device. In other embodiments, the ablation probe may be used with other types of intraluminal delivery device.

The ablation probe extends between a proximal end 100a and a distal end 100b. The terms "distal" and "proximal" are taken relative to the user operating the ablation probe and the treatment site when the ablation probe is positioned for use - the distal end 100b of the ablation probe 100 is that closest to the treatment site and the proximal end 100a is that closest to the user. The ablation probe 100 has a longitudinal axis Y that extends the length of the device between the distal and proximal ends. A radial direction R is defined as a direction normal to the longitudinal axis Y as shown in Figure 1. A reference to being along the "length" of the ablation probe or other component herein refers to the length in a direction along or parallel to the longitudinal axis Y.

A handle 104 is provided at the proximal end of the ablation probe 100 so that it can be manipulated and positioned by the user. The distal end 100b is shown in Figure 1 after being fed through the working channel 102 of an endoscope or similar device to reach a target ablation site, with the distal end 100b and a portion of the length of the ablation probe (i.e. a antenna region) extending out of the distal end of the working channel.

The ablation probe 100 generally comprises an applicator 106 and a feeding or feed cable 108. The terms "feeding cable" and "feed cable" are used interchangeably herein. The applicator 106 comprises an antenna and is arranged to emit radiation to heat surrounding tissue when the ablation probe 100 is in use. The emitted radiation may be used to cause localised heating and destruction of malignant cells around or near to the applicator 106. The applicator 106 may be arranged to apply any suitable form of radiation to surrounding tissue such that the desired heating is caused. The applicator 106 may, for example, be arranged to emit microwave or RF radiation, or may emit any other suitable radiation to cause heating. The applicator 106 is arranged at or near a distal end of the ablation probe 100 so that it can be positioned in a desired position relative to the tissue to be treated.

The feed cable 108 is arranged to supply electromagnetic energy to the applicator 108. Only part of the length of the feeding cable 108 is shown in the Figures. The feed cable 108 may be an elongate member suitable for supplying electromagnetic energy to the applicator (e.g. a conductor), but may include additional components (e.g. coolant conduits). The feed cable 108 may run along at least part or all of the length of the ablation probe 100 to deliver a supply of energy to the applicator 106. In the described embodiment, the applicator 106 is located at the distal end of the feed cable 108 and a proximal end of the feed cable 108 is coupled to a generator (not shown in the Figures) suitable for generating the desired frequency signal to supply energy to the applicator 106. In the presently described embodiment, the feed cable 108 comprises a coaxial cable as will be described in more detail later. An additional length of coaxial cable not shown in the figures may be used to connect the distal end of the feed cable (e.g. at the handle 104) to the signal generator. Similar additional connections may be provided for a coolant circuit which are also not shown in the Figures.

The ablation probe 100 further comprises a tubular member 110 which is arranged to house the feed cable 108. The tubular member extends between a proximal end at which it is connected to the handle 104 and a distal end which is inserted through the working channel 102 to reach the ablation site. The tubular member 110 is sufficiently flexible such that it can extend along a tortuous path through the patient's anatomy. The tubular member 110 may have a generally circular cross section to match that of the feed cable 108, but other shapes are possible. The tubular member 110 may be referred to as a catheter tube.

The tubular member 110 comprises a shaft portion 111 and a tip portion 112 located at its distal end as can be seen in Figure 1. The shaft portion 111 and the tip portion 112 define a hollow space in which the feed cable 108 and applicator 106 are located. The tip portion 112 forms a pointed tip of the ablation probe 100 that is suitable for piercing tissue in use. In the present embodiment, the tip portion 112 comprises a separate component that is bonded to the shaft portion 111 as will be described later. In other embodiments, a separate tip component may not be provided, such that the tip portion is formed integrally with the shaft portion 111. In some embodiments, the tip 112 portion may have an atraumatic shape, rather than a pointed one.

In the described embodiment, the feed cable (and surrounding tubular member 110) have an approximately constant shape and cross section along their length between the distal and proximal ends of the ablation probe.

In other embodiments, the feed cable 108 (and tubular member 110) may have one or more sections having a different shape and/or cross-sectional size. In some embodiments, the ablation probe 100 generally comprises two portions: a needle portion and a catheter portion. The needle portion may be arranged at the distal end of the ablation probe 100 and is adapted to be inserted into tissue during use to reach the desired ablation location. The catheter portion may be provided at the proximal end of the ablation probe 100 and is arranged to supply electromagnetic energy to the needle portion (and optionally a flow of coolant to and from the needle portion 100). The catheter portion may have an extended length and flexibility for endoscopic use. The needle portion may include a section of the feed cable 108 which has a smaller cross section compared to that of the catheter portion. This may help to reduce the need for cooling of the feed cable along the catheter portion.

In some embodiments, the needle portion may form a small part of the overall length of the ablation probe. For example, the needle portion may be 5 mm to 2000 mm in length, and preferably may be around 70 mm in length. The length of the needle portion may be chosen according to the anatomy to be accessed. For example, the needle portion may be approximately between 10 and 100 mm long for delivery of therapy to organs including the pancreas, or lung, or longer (for example 100-400 mm in length) for delivery of therapy percutaneously. A longer length of needle portion may be more suitable for accessing parts of the lung, for example. The catheter portion may be around 1000 mm to 2000 mm in length, and preferably around 1400 mm in length. The length of the catheter portion may be chosen according to the position of the ablation site which must be reached.

Referring again to Figure 1, the ablation probe 100 further comprises a restriction means or arrangement 114 (i.e. one or more restriction/restricting features) which is arranged to restrict or control the separation X between the applicator 106 and the tubular member tip portion 112. More specifically, the restriction means 114 is arranged to restrict the separation between a point on the applicator 106 corresponding to the centre of the ablation zone it produces and a point at the most distal extent of the tip portion 112. That distance is labelled X in the Figures. The centre of the ablation zone corresponds to the feed point of the applicator as will be described later. The restriction means is arranged to control the separation of these two points during use of the ablation probe 100 so that they have a restricted range of relative movement between them (which may be zero movement or close to zero movement). The restriction means is therefore arranged to provide a mechanical engagement between the distal part of the applicator and a proximal part of the tip portion 112 to control relative movement between them.

To allow the ablation probe to bend during use, the feed cable 108 is otherwise free to move relative to the tubular member 110. In some embodiments, there may be no rigid connection between the tubular member 110 and the feed cable 108 (apart from an indirect coupling via the restriction means as will be described later). More generally, at least a distal section (e.g. the majority) of the length of the tubular member 110 between its distal and proximal ends is otherwise movable (e.g. proximally and distally) relative to the corresponding section of the feed cable 108 which it surrounds. In such embodiments, there may be one or more rigid connections between the feed cable 108 and tubular member 110 spaced between the distal and proximal ends of the ablation probe 100. For example, there may be a rigid connection at a point where there is a change in size of the feed cable or rigid connections formed by spacer components used to form coolant channels between the feed cable and tubular member.

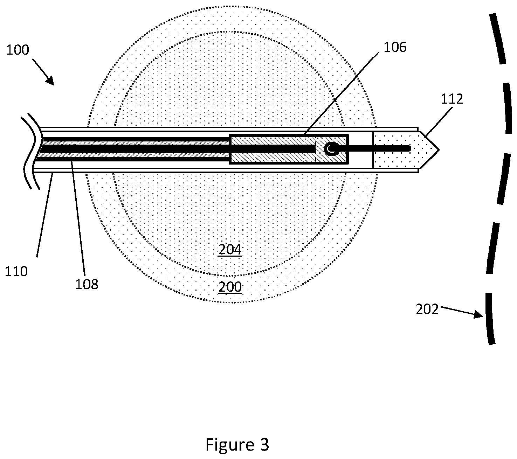

The effect of the restriction means 114 is illustrated in Figures 2a, 2b and 3. Figures 2a and 2b show an example of an ablation probe without the restriction means of the present application. The same reference numbers used in Figure 1 have been used in Figures 2a and 2b to aid explanation. In Figure 2a the ablation probe is in a straight configuration. Figure 2b shows the same ablation probe after having been bent to reach an ablation site. In Figure 2a, corresponding points A and B along the respective length of the feed cable 108 and tubular member 110 are aligned. In Figure 2b, points A and B have become misaligned along the length of the ablation probe because of the change in shape of the feed cable 108 and tubular member 110 and because of their freedom to move relative to each other. Where these components are made from different materials, they may have different bending properties. This means that they will not change shape in the same way when the ablation probe is extended along a tortuous path through the patient's anatomy. The bending of the ablation probe therefore causes a resulting movement of applicator 106 relative to the tip portion 112 as the feed cable moves within the tubular member. This can be seen in Figures 2a and 2b by a change in the length X. The same movement would be present in an ablation probe of the present invention, but the range of movement X is restricted by the restriction means 114.

The inventors have determined that controlling the separation between the applicator 106 and the distal tip portion 112 of the ablation probe has several advantages. These can be best understood by reference to Figure 3, which shows an embodiment of the present application. In Figure 3, the ablation probe 100 is illustrated positioned within a lesion (e.g. tumour) 200 that is to be ablated. The lesion 200 is located close to a critical anatomical feature 202 (pleura, large blood vessel, large airway etc). The clinician must align the centre of an ablation zone 204 created by the applicator 106 with the centre of the lesion 204 and ensure the tip 112 of the ablation probe is placed at a safe distance from the critical anatomical structure 202. This ensures that the ablation zone 204 is properly aligned with the lesion 200 to reduce the ablation of healthy tissue while also avoiding damaging critical structures 202. By controlling the separation between the applicator 106 and distal tip 112 the clinician is able to locate them relative to each other when carrying out the procedure. For example, if one of the applicator or distal tip are visible under image guidance the position of both of them will be known as described later.

Advantageously, the separation between the proximal end of the applicator (i.e. the point at which it connects to the outer conductor of the feed cable) and the most distal point of the tip is controlled. This is advantageous as the distal end of the applicator is typically at the centre of the ablation zone and the most distal point of the tip is the part of the ablation probe which would make first contact with the critical structure 202.

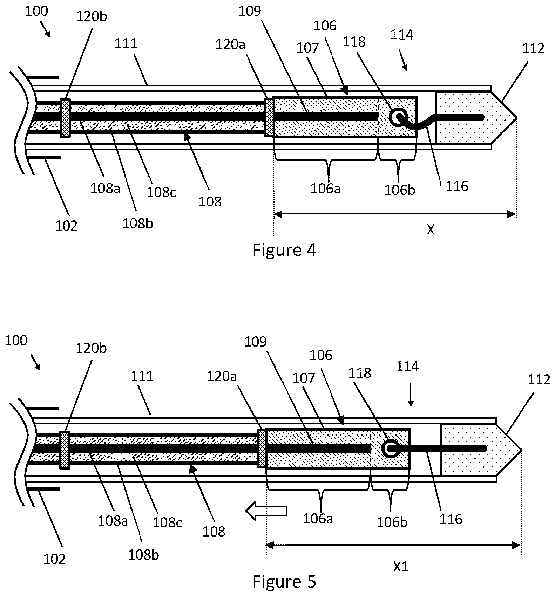

Further details of the restriction means 114 are shown in Figures 4, 5 and 6. These Figures each show a close-up view of a distal region of the ablation probe shown in Figure 1.

The restriction means 114 is arranged to limit proximal movement of the applicator 106 relative to the tip portion 112 of the tubular member 110. In the present embodiment, the restriction means 114 comprises a tethering member 116 arranged to limit the range of proximal movement of the applicator 106 relative to the tip portion 112. The tethering member 116 comprises an elongate flexible coupling member extending between the applicator 106 and the tip portion 112 of the tubular member 110. In the present embodiment, the flexible coupling member comprises a filament.

The function of the tethering member 116 can best be understood from Figures 4 and 5. The tethering member 116 comprises a flexible length of material that is coupled between the applicator 106 and the tip portion 112. Movement of the applicator 106 proximally relative to the tip portion 112 (e.g. in the direction of the arrow in Figure 5) causes tension in the tethering member 116. This restricts the range of movement of the applicator 106 in a proximal direction relative to the tip portion 112. The length of the tethering member 116 therefore sets a maximum separation X1 between a point on the applicator 106 and a point on the tip portion 112 (e.g. sets a maximum valve of the separation X marked in Figure 1 and defined above).

In the presently described embodiment, the tethering member 116 comprises a polymer material. In some examples, the tethering member 116 may be formed from (i.e. may comprise) Fluorinated ethylene propylene (FEP). In other examples, other materials may be used for the tethering member.

More generally, the tethering member 116 (e.g. the filament) may comprise or be made from an electrically insulating material. This may help to avoid electromagnetic interference with the applicator and overheating. The tethering member 116 may be arranged to preserve its mechanical properties at high temperatures, which may allow it to withstand the temperatures that may be experienced during an ablation procedure.

As can be seen in Figures 4 and 5, the tethering member 116 is embedded into the tip portion 112 such that part of its length extends into the body of the tip portion. This may provide a secure bond between them. In some embodiments, the tethering member may be formed from the same material as the tip portion 112 (i.e. they may both comprise the same material). For example, they may both comprise the same polymeric material. More specifically, they may both comprise FEP. By using the same material for the tip portion 112 and tethering member 116 a stronger bond may be formed between them.

In some embodiments, the tethering member 116 may be reflow bonded to the tip portion 112. For example, the material of the tethering member 116 and tip portion 112 may be heated to above their melting temperature and allowed to mix and cool to form a bond between them. By bonding the tethering member 116 and tip portion 112 in this way a secure bond may be formed. In other embodiments, other bonding techniques may be used. The tethering member 116 and tip portion 112 may be bonded together using an adhesive, for example.

The tethering member 116 described above provides advantageous restriction of the range of movement of the applicator 106 relative to the tip portion 112. This may give the physician using the ablation probe a fixed reference distance between the ablation zone and the tip's location within the anatomy of the patient. Moreover, by using a flexible coupling component to connect the tip portion 112 and applicator 106 the overall flexibility of the ablation probe is not inhibited. If the applicator 106 were bonded directly to the tip portion 112 instead, the flexibility of the applicator region of the ablation probe would be reduced and tight bend radii may no longer be accessible.

The filament used to form the tethering member 116 is advantageous in that it provides good flexibility and ease of manufacture. In other embodiments, the tethering member 116 may take other forms. For example, the tethering member 116 may be formed from other types of elongate flexible components, such as a spring. In other embodiments, the tethering member 116 may be any suitable component which can provide flexibility and sufficient strength to limit the extent of the distal movement of the applicator 106 relative to the tip 112.

The flexibility of the tethering member 116 means that it provides little or no resistance to distal movement of the applicator 106 relative to the tip 112. As can be seen in Figure 4, the tethering member 116 may bend or flex such that the applicator 106 is relatively free to move towards the tip portion 112.

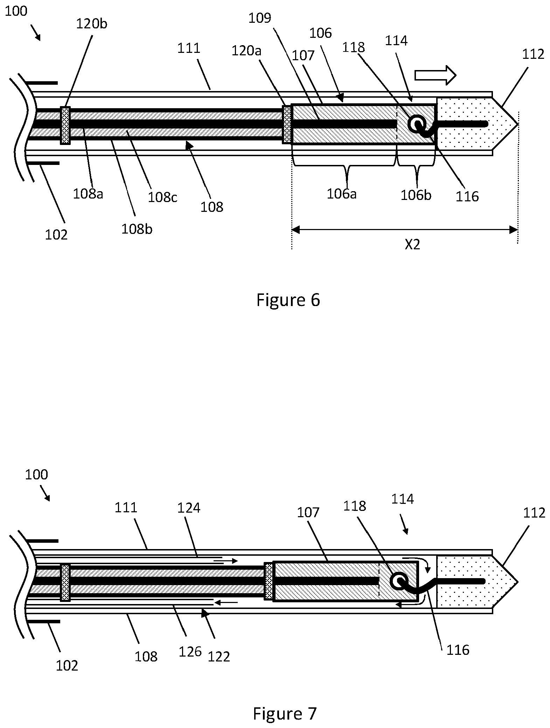

The restriction means 114 is therefore further arranged to limit distal movement of a radiating section of the antenna relative to the tip of the tubular member. This is illustrated in Figure 6. The distal movement of the applicator is in the direction shown by the arrow in Figure 6.

As can be seen in Figures 4, 5 and 6, the applicator 106 comprises an electrically conducting straight antenna section 109 and a dielectric resonator 107. The straight antenna section 109 is in electrical communication with the feed cable 108. The dielectric resonator 107 is formed from a dielectric material such as a ceramic. The feed cable 108 comprises a coaxial cable which comprises an inner conductor 108a, and outer conductor 108b and a dielectric 108c between them. In the presently described embodiment, the straight antenna section 109 comprises a portion of the inner conductor 108a of the feed cable 108 extending into the dielectric resonator 107 of the applicator 106.

The portion of the applicator in which the antenna member 107 is located forms a radiating section 106a of the applicator 106. The radiating section is the part of the applicator 106 which acts as an antenna for the emission of radiation (e.g. microwave radiation). The straight antenna section 109 shown in the figures forms a monopole antenna. In other embodiments, other types of antennae may be used. For example, the straight antenna section 109 may be coupled to or replaced with a helical shaped antenna component rather than being formed from only a straight length of the inner conductor as will be described in more detail later. In other embodiments, the straight antenna section 109 may comprise a separate component electrically connected to the inner conductor 108 rather than being integral with it.

The radiating section 106a of the applicator 106 may be defined as the length of the applicator which is arranged to emit radiation as illustrated in Figure 6. In the present embodiment, that is the length of the applicator in which the straight antenna section 109 extends. More specifically, it may be the length of the applicator 106 between the most distal extent of the outer conductor 108b of the feed cable and the most distal extent of the straight antenna section 109 (e.g. the most distal extent of the inner conductor 108a).

The restriction means 114 is arranged to limit the movement of the radiating section 106b in a distal direction towards the tip portion 112. In other words, the restriction means 114 is arranged provide a minimum spacing (which is greater than a zero spacing) between the most distal point of the radiating section 106a and the tip portion 112. As the applicator 106 is formed from a relatively rigid material, this provides a minimum of X2 for the range of the distance X between the most proximal end of the radiating section 106a and the most distal extent of the tip portion 112 as shown in Figure 6.

By limiting the distance that the radiating section 106a can move towards the tip portion 112 the risk of the antenna detuning may be reduced. If the antenna member 106a moves too close to the tip portion 112 of the ablation probe 100 it may detune, which may result in delivery of a suboptimal energy dose to the tissue being ablated.

To restrict the extent of the distal movement of the radiating section 106a, the restriction means 114 comprises an abutment portion 106b of the applicator 106. The abutment portion 106b extends distally between the radiating section 106a of the applicator 106 and the most distal extent of the applicator 106. This means that proximal movement of the applicator 106 towards the tip portion 112 (e.g. in the direction of the arrow in Figure 6) results in the abutment portion 106b contacting the tip portion 112. The abutment portion 106b therefore provides a minimum separation between the radiating section 106a and the tip portion 112. The abutment portion 106b is advantageous in that it allows a minimum separation between the radiating section 106a and the tip 112 to be set, while still allowing flexibility and pushability of the ablation probe.

The restriction means 114 may restrict the range of movement of the applicator relative to the tip portion (e.g. the range of distance X may be restricted to the range X2 to X1). In some embodiments, that range may be small or effectively zero if the tethering member 116 is short enough so that it is in tension with the abutment portion close to or contacting the tip portion.

Referring again to Figures 4, 5 and 6, the abutment portion 106b comprises an anchor point 118 for the tethering member 116. In the presently described embodiment, the anchor point comprises a hole in the abutment portion 106b arranged to couple with the tethering member 116. For example, the filament forming the tethering member 116 may be coupled using the hole e.g. it may be tied through the hole to secure the applicator 106 and tethering member 116 together. This may aid manufacture. In other embodiments, other types of coupling between the tethering member 116 and anchor point 118 may be provided. For example, the anchor point may be a location at which the tethering member 116 is glued to the applicator.

The ablation probe 100 may comprise one or more markers forming a visual indicator allowing the user to determine the position of the ablation probe during use. The marker(s) may be a radiopaque marker(s). By a radiopaque, we mean that they are opaque to one or another form of radiation, more specifically is opaque to X-ray radiation. They are therefore visible using imaging (e.g. X-ray imaging) during use of the ablation probe 100. In other embodiments, the marker(s) may be echogenic markers. Such markers may be imaged using ultrasound. In yet other embodiments, other kinds of marker may be used that are visible using any suitable type of imaging technique.

The one or more markers may be one or more separate components provided as part of the ablation probe 100 to act as markers. For example, the markers may comprise a band of material (e.g. a metallic band) that extends around part of the ablation probe so that it is secured in position. Additionally, or alternatively, the marker or markers may be a component or components of the ablation probe made from an indicator material (e.g. a material visible using a suitable imaging technique e.g. a radiopaque and/or echogenic material).

The one or more markers may be located at a known fixed position relative to the centre of the ablation zone produced by the applicator 106. In the present embodiment, the centre of the ablation zone corresponds to the feed point of the applicator 106. The feed point may correspond to the most distal point along the length of the ablation probe 100 at which the inner conductor 108a is first exposed outside of the outer conductor 108b (i.e. the point just past the point at which the outer conductor extends no further along the longitudinal length of the ablation probe).

In use, the marker(s) may be used to locate the centre of the ablation zone so that it can be accurately positioned within the anatomy of the patient. By using the restricted range of movement between the applicator 106 and the tip portion 112 provided by the restriction means 114, the position of the tip portion may also be inferred and used to avoid contacting critical anatomical structures with the tip (e.g. as shown in Figure 3).

Additionally, or alternatively, one or more of the markers may be located at a known fixed position relative to the most distal point of the tip portion 112. This may allow the tip portion 112 to be positioned without damaging critical tissue. The position of the ablation zone may be inferred from the restricted range of movement provided by the restriction means 114 and the ablation zone positioned accurately while also positioning the tip to avoid critical structures.

One, or both, of the tip portion 112 and applicator 106 may comprise an indicator material visible under imaging to form a marker having a known fixed position relative to the centre of the ablation zone or the distal end of the tip respectively. This may allow the position of both of those points to be determined during use. The tip portion and/or the applicator may therefore act as one of the one or more markers.

In some embodiments, the applicator 106 may comprise a marker formed by a marker band which extends around the dielectric resonator 107. In some embodiments, further marker bands may be provided. In other embodiments, any other suitable marker may be included as part of the applicator, for example a radiopaque/echogenic coating or other component may be added to the applicator 106.

In other embodiments, additionally or alternatively, the dielectric resonator 107 may comprise an indicator material. For example, the dielectric resonator may be formed from a radiopaque ceramic material. This may provide a suitable marker without needing to add a further marker component. Additionally, or alternatively, the dielectric resonator 107 may be made from an echogenic material.

In some embodiments, the tip portion 112 may be formed from an indicator material. For example, the tip portion 112 may be formed from a polymeric material which has been doped with an indictor material. The indicator material may be a radiopaque material (e.g. barium sulphate). Other materials may be used. In some embodiments, the tip portion 112 may comprise a radiopaque/echogenic section of the tubular member 110. It may, for example, comprise a section of the tubular member 110 which has been doped with an indicator material (e.g. barium sulphate). In other embodiments, the tip portion 112 may comprise a separate marker component. For example, a marker band may extend around the tip portion 112 to allow its position to be located.

In the embodiment shown in Figures 4 to 7, the ablation probe 100 comprises a first marker 120a located at or near to the centre of the ablation zone. In the presently described embodiment, the first marker is located on the feed cable 108. For example, the first maker 120a is located adjacent the point at which the feed cable 108 connects to the dielectric resonator 107.

The ablation probe 100 further comprises a second marker 120b. The second marker 120b is also located on the feed cable 108. The second marker 120b is arranged to indicate a safe distance for the distal end of the working channel 102 away from the ablation zone. For example, the second marker may be located a distance from the expected centre of the ablation zone at which the level of radiation emitted by the applicator has reduced to a threshold safe level. This may allow the ablation probe to be extended out of the working channel sufficiently to avoid damage to the deliver device when the ablation is activated. The second marker 120b may be located about 1 cm from the margin of the ablation zone in its largest operating setting (i.e. the largest size of ablation zone that can be produced by a specific implementation of the ablation probe).

The first and second makers 120a, 120b may each comprise a band fixed around the feed cable. The band may be any suitable indicator material, such as a metal band.

In some embodiments, the ablation probe 100 may comprise a coolant circuit 122 as illustrated in Figure 7. The coolant circuit may be arranged to carry a flow of coolant along the length of the ablation probe 100 from the proximal end to the applicator 106 and back again to provide cooling of the applicator 106 and other components. The coolant circuit 112 may be connected to a suitable coolant pumping system via the handle 104. The coolant may be water or any other suitable coolant.

As can be seen in Figure 7, the coolant circuit 122 comprises a first coolant conduit 124 arranged to carry a flow of coolant in a distal direction along the length of the ablation probe 100 and a second coolant conduit 126 arranged to carry a flow of coolant in the opposite direction. The coolant may flow out of the first coolant conduit 124, around the applicator 106 (or around at least part of it) and return along the second coolant channel 126. In the described embodiment, the coolant conduits 124, 126 are formed by separate components located between the feed cable 108 and tubular member 110. Other suitable structures may be used to carry coolant. For example, one or both of the coolant conduits 124, 126 may be formed by the space between the feed cable 108 and tubular member 110. In some embodiments, more than one coolant conduit may be provided to carry coolant in each direction along the ablation probe 100.

The coolant circuit 122 is arranged to extend through the restriction means 114 so that coolant can flow around the distal end of the applicator 106. In some embodiments, the coolant may therefore be allowed to flow around all of the applicator. This may improve the cooling of the applicator 106 and improve the stability of the ablation probe. By using the restriction means 114 of the present application the freedom of movement of the applicator 106 relative to the tip portion 112 may be restricted while maintaining effective cooling of the applicator 106. The use of the tethering member 116 may be advantageous in allowing coolant to flow around it and so around the applicator 106. For example, the elongate shape of the tethering member 116 may allow sufficient flow of coolant through the restriction means 114 (e.g. compared to if there were a rigid coupling between the tip 112 and applicator 106).

The restriction means generally comprises a structure to limit the range of movement of the applicator 106 relative to the tip portion 112. In the previously described embodiments, the restriction means comprises separate structures to limit the range of proximal movement (e.g. the tethering member 116) and the range of distal movement (e.g. the abutment portion 106b) of the applicator 106 relative to the tip portion 112. In other embodiments, only one of these functions may be provided. In yet other embodiments, a single structure may provide both functions. For example, a single flexible coupling may extend between the applicator 106 and the tip portion 112. Such a flexible coupling may have sufficient strength to resist both proximal and distal movement of the applicator relative to the tip.

In the embodiments shown in Figures 1 and 3 to 7 the ablation probe comprises only a straight antenna section 109 extending within the dielectric resonator 107 to form the antenna of the ablation probe. In other embodiments, a different antenna geometry may be used. For example, in any of the embodiments described herein a helical antenna may be used.

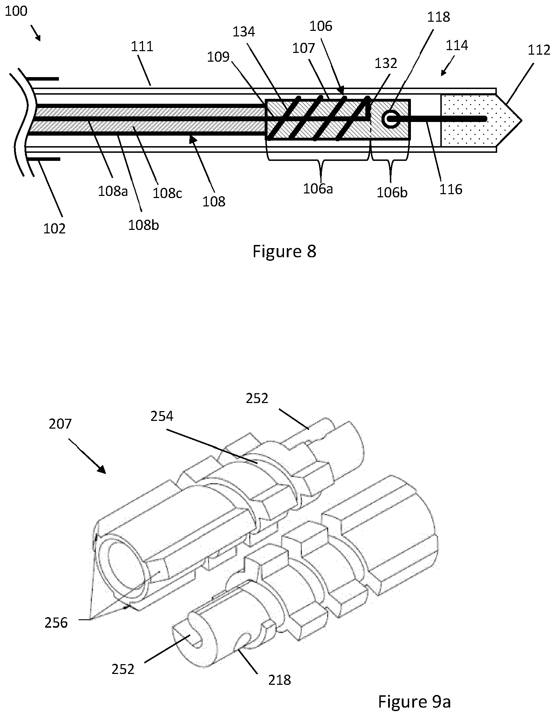

Figure 8 shows an embodiment of the ablation probe 100 which comprises a helical antenna. In this embodiment, the applicator 106 comprises a radially extending antenna section 132 and a helical antenna section 134 in addition to straight antenna section 109. The straight antenna section 109 is electrically connected to the helical antenna section 134 via the radially extending antenna section 132. The antenna sections 109, 132, 134 are all formed from part of the inner conductor 108a in the presently described embodiment. In other embodiments however, they may be formed from separate conducting elements connected together.

Any of the features described in connection with Figures 1 to 7 may also apply to the embodiment of Figure 8 (i.e. it may include markers and a coolant circuit)

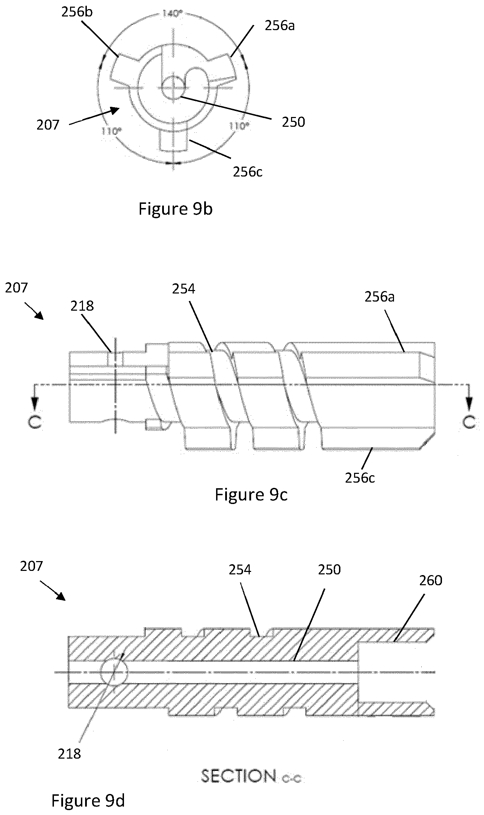

An example of a dielectric resonator 207 which can be used with any of the embodiments described herein is shown in Figures 9a-d. Figure 9a shows perspective views of the dielectric resonator 207 from two different angles, Figure 9b shows an end view, Figure 9c shows a side view and Figure 9d shows a cross section through plane C-C.

The dielectric resonator 207 is adapted for use with a helical antenna similar to that shown in Figure 8. The dielectric resonator 207 comprises an axial hole 250 (a through-hole in the present example) configured to receive the straight antenna section 109 of the antenna. The dielectric resonator 207 further comprises a radial hole 252 configured to receive the radially extending section 132 of the antenna. In the present embodiment, the radial hole 352 comprises a slot extending in a radial direction through the body of the dielectric resonator 207 such that it meets the axial hole 250. Other arrangements of radial hole 252 may be used, however.

The dielectric resonator 207 further comprises a helical channel (e.g. a groove) 254 arranged to receive the helical section 134 of the antenna. The helical channel 254 is generally formed on the outer surface of the dielectric resonator 207. The helical channel 254 is arranged to retain the helical section 134 of the antenna and maintain it in the desired helical shape. This ensures that the geometry of the helical section 134 remains constant during use of the ablation probe and reduces the risk of the antenna detuning. In some embodiments, the helical channel 254 may be absent. In such embodiments, the helical section of the antenna may be a coating applied to the outer surface of the dielectric resonator 207 or may be fixed using an alternative method such as adhesive. Although a continuous helical channel is shown in the Figures, the channel may more generally extend along a helical path in one or more sections.

More generally, the dielectric resonator 207 may comprise one or more retention features on its outer surface to retain a section of the antenna. The helical groove is one such example of a retention feature. In other embodiments, the retention feature may comprise a groove having a different shape, or other protrusion or interlocking feature which can engage with the antenna and resist it being pulled away from the applicator.

In addition to maintaining the helical shape of the antenna, the engagement of the antenna sections with the radially extending hole 352 and helical channel 354 provides a coupling between the part of the inner conductor forming the antenna and the dielectric resonator 207. This allows the dielectric resonator 207 to retain (e.g. form a connection with, resist them from pulling apart) the inner conductor (and hence the feed cable) without the need for other fixing means, such as adhesive. This improves structural strength of the ablation probe and aids manufacture.

Referring again to the figures, the dielectric resonator 207 further comprises a plurality of fins 256. As can be seen more clearly in Figure 9b, the dielectric resonator 207 comprises three fins 256a, 256b, 256c formed on its outer surface. The fins 256 are configured to abut an inner surface of the tubular member 110 such that the dielectric resonator 207 and tubular member 110 are aligned concentrically. The helical channel 254 extends through the fins 256 to form a continuous channel around the dielectric resonator as can be seen in Figure 9a. In other embodiments, the helical channel 254 may be formed from broken sections - i.e. it may be formed from cuts in the fins which are arranged to retain the helical section of the antenna in a helical geometry. The channel therefore extends along a helical path around the longitudinal axis of the ablation probe.

Although three fins are shown in Figure 9a-d there may be greater than or less than three. Preferably there may be at least three fins to maintain concentricity of the dielectric resonator 207 and tubular member 110. The fins may be unevenly spaced about the circumference of the dielectric resonator 207 as shown in Figure 9b. In other embodiments, they may be evenly spaced. The spacing shown in Figure 9b is only one example.

Referring to Figure 9d, the dielectric resonator 207 further comprises an aperture 260 configured to receive the exposed portion of the dielectric 108c and inner conductor 108a of the feed cable. The aperture 260 is formed in the present embodiment by an axial bore formed in the proximal end of the dielectric resonator 207. The aperture 260 is axially aligned with the axial hole 250 as shown in Figure 9d. By inserting the exposed portion of the dielectric and inner conductor in this way a flexible connection between the dielectric resonator and feed cable can be formed.

This may aid the ablation probe in bending to navigate a tortuous route through the anatomy of the patient.

As can be seen in Figure 9d, the dielectric resonator 207 comprises an anchor point 218 for the tethering member 116 as previously described. The anchor point 218 comprises a through-hole extending radially though the dielectric resonator.

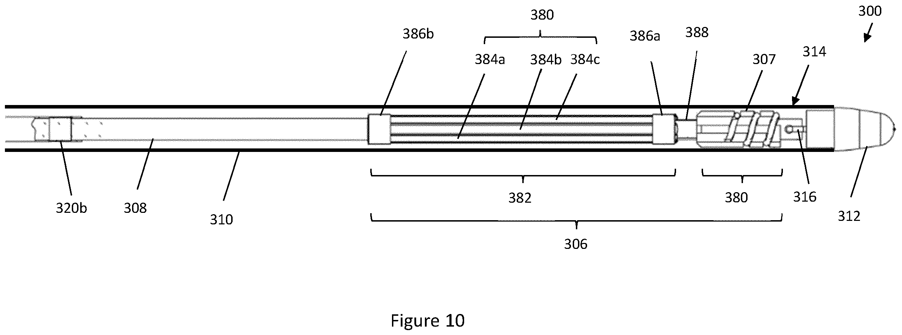

Figure 10 shows another embodiment of an ablation probe 300. Corresponding reference numbers have been used to those used in Figures 1 to 8 for features common between those embodiments. The ablation probe 300 generally comprises an applicator 306, feed cable 308, tubular member 310, tip portion 312 and restricting means 314 similar to those described above. Figure 10 shows a cut-away side view in which the components internal to the tubular member 310 are visible. The ablation probe 300 may include any of the features described in connection with any other embodiment disclosed herein.

In the embodiment shown in Figure 10, the applicator 306 comprises a dipole antenna rather than a monopole antenna. The dipole antenna comprises a first antenna section 380 forming a first pole of the antenna and a second antenna section 382 forming a second pole of the antenna.

The first antenna section 382 comprises a helical antenna as described above. The applicator 306 therefore comprises a dielectric resonator 307 that corresponds to that shown in Figures 9a-d. In other embodiments, the first antenna section may comprise a straight antenna conductor similar to that described above in connection with Figures 1 to 7.

The second antenna section 382 is located proximally along the length of the ablation probe 300 relative to the first antenna section 380. The second antenna section 382 comprises a plurality of antenna elements 380 (or antenna members). Three such antenna elements 384a, 384b, 384c are visible in Figure 10. The applicator may comprise any suitable number of antenna elements 380.

The feed cable 308 comprises an inner conductor, outer conductor and dielectric similarly to that of the embodiment shown in Figures 1 to 7. Each antenna element 380 is formed integrally with the outer conductor of the feed cable 308. The antenna elements are formed from part of the outer conductor which is bent-back such that it extends back proximally along the length of the feed cable 308. Each of the antenna elements 380 therefore comprises an outwardly extending portion that extends in a direction outwards away from the longitudinal axis of the ablation probe e.g. in a direction having a component in a radial direction R away from the longitudinal axis Y of the ablation probe (which corresponds to the longitudinal axis of the coaxial cable). Each antenna element 380 further comprises an overlapping portion extending proximally along the length of the coaxial cable (e.g. in a direction parallel to the longitudinal axis Y). The radial portion and the overlapping portion are formed integrally with the rest of the outer conductor i.e. they are a continuous piece of material with no joints (such as solder joints or welds) between them. The outwardly extending and overlapping portions are formed by shaping (e.g. bending) a part of the outer conductor so that it extends away from and back along the coaxial cable so that part of the dielectric is exposed.

The second antenna section 382 further comprises an antenna insulator (not visible in the figures) arranged to separate or space apart the overlapping portion of each antenna element from the part of the outer conductor 308 which it overlaps. The antenna elements 380 may lie on the outside surface of the insular material. In other embodiments, they may be embedded into the insulator material, or located in recesses in its surface. The antenna insulator is arranged to electrically insulate the overlapping portions from the adjacent part of the outer conductor 308.

In the presently described embodiment, the outer conductor of the feed cable is a braided outer conductor. The antenna elements 380 are formed from an un-braided part of the outer conductor. For example, each antenna element may be formed by one of the wire strands of the un-braided part of the outer conductor. This allows the antenna elements to be formed by un-braiding (which includes at least partially un-braiding) the separate strands of the outer conductor such that they are free of each other and shaping them (e.g. bending them) so that they extend away from the outer conductor and proximally back along the cable. This allows the antenna elements to be manufactured integrally with the outer conductor. In other embodiments, the outer conductor can be un-braided and the wire stands forming it used in any other way to create the antenna elements e.g. one or more wire strands forming the braided conductor may be used to form each antenna element, or separate wire filaments forming each strand may be un-braided to form one or more antenna elements. In some embodiments, the wire strands of the outer conductor which form the antenna elements may be re-braided so that they are woven together. For example, a portion of the outer-conductor may be un-braided and then the un-braided stands forming each antenna element re-woven or re-braided into a new braided section of the outer conductor.

In other embodiments, the outer conductor comprises a solid conducing sheath or tube of material surrounding the dielectric rather than braided wire strands. The outer conductor is therefore a single solid element, rather than made of individual strands. In that case, the outer conductor may be cut to form a series of strips of material. Each strip may then be shaped (e.g. bent) to form the outwardly extending portions and overlapping portions of each antenna element 380.

The second antenna section 382 further comprises one or more retaining bands. In the described embodiment, a first and second retaining band 386a, 386b are provided, but there may be only one or more than two in other embodiments.

The retaining bands 386a, 386b are arranged to secure the antenna elements 380 around the antenna insulator. The retaining bands may be made from an indicator material that is visible under imaging (e.g. a radiopaque and/or echogenic material). They may therefore act as the markers (or act as some of the markers) described elsewhere herein. For example, the first retaining band 386a may be used to mark the centre of the ablation zone.

As can be seen in Figure 10, the applicator 306 comprises a flexible hinge region 388. A proximal end of the dielectric resonator 307 is spaced apart from the most distal extent of the outer conductor of the feed cable along the length of the feed cable 308 to form the hinge region 358 between them.

As can be seen in Figure 10, the proximal end of the dielectric resonator 307 is spaced apart from the antenna elements 380 along the length of the coaxial cable. The hinge portion is therefore formed from a section of the coaxial cable where the dielectric layer of the cable is exposed (but where it is not inserted into the dielectric resonator).

The hinge region 388 comprises a region of greater flexibility compared to the part of the antenna where the outer conductor is present and compared to the dielectric resonator 307. This provides a point of preferential bending of the ablation probe away from where components are connected together (e.g. where the dielectric resonator is connected to the inner conductor). This may help to avoid those connections from failing when the ablation probe is bent during use.

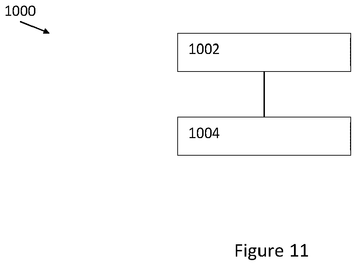

The present application further provides a method 1000 of manufacturing the ablation probe of any embodiment described herein. The method 1000 is illustrated schematically in Figure 11.

Manufacturing 1000 the ablation probe comprises a step of bonding 1002 the tethering member 116 to the tip portion 112 of the tubular member 110. The bonding step 1002 comprises reflowing the material of the tethering member 116 and tip portion 112 to join them together. This may be achieved by heating the two components, inserting the tethering member 116 into the tip portion 112 and allowing them to cool to form a bond between them. The components may be heated to their melting point or above (or to the glass transition temperature or above) to bond them together. To bond the tip portion 112 and tethering member 116 together by reflowing they may be both made from the same material as described above. Preferably, this may be a polymeric material (preferably FEP).

Manufacturing 1000 the ablation probe further comprises a step 1004 of making the tip portion 112 visible using image guidance. This may comprise making the tip portion radiopaque and/or echogenic. In the present embodiment, making the tip portion 112 visible under image guidance comprises reflowing the tip portion 112 with an indicator material. As a result, the tip portion also will also have the same properties.

To make the tip portion visible using image guidance, the method 1000 comprises reflowing the material of the tip portion 112 with a material which has been doped with an indicator material. For example, the tip portion 112 may be reflowed with a polymer material (such as FEP) which has been doped with a radiopaque material (e.g. barium sulphate) or an echogenic material.

The steps of bonding the tethering member 116 to the tip portion 112 and making the tip portion 112 visible under image guidance may be combined into a single operation (e.g. they may occur simultaneously). For example, the tip portion 112 and tethering member 116 may be reflowed together and with an indicator material in a single operation to both bond them together than make the tip portion radiopaque. In some embodiments, the tip portion 112, tethering member 116 and indicator material may comprise the same material to allow them to be more easily be reflowed together. They may, for example, all comprise a polymer material such as FEP.

The method 1000 may further comprise bonding a tip component and shaft portion to form the tubular member (e.g. where by the tip portion is not integral with the shaft portion of the tubular member). The tip component may be bonded using a reflow process. The tip component may be bonded in a single reflow process in which the tethering member is also bonded to the tip component and/or in which the tip is also made visible under image guidance.

The skilled person will understand that further steps are provided in the method 1000 to assemble the various components an ablation probe of any of the embodiments described or claimed herein. Those additional steps are not described in any further detail in this application.

Various modifications will be apparent to the skilled person without departing form the scope of the claims. Any feature disclosed in connection with one embodiment may be used in combination with the features of another embodiment.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness, it is also stated that the term "comprising" does not exclude other elements or steps, the term "a" or "an" does not exclude a plurality, a single processor or other unit may fulfil the functions of several means recited in the claims and any reference signs in the claims shall not be construed as limiting the scope of the claims.

## Claims

1. A microwave ablation probe, comprising:
- an applicator having a radiating section arranged to emit electromagnetic energy to cause heating in tissue;
- a feed cable having a distal end and a proximal end and being arranged to supply electromagnetic energy to the applicator, the applicator being coupled to the distal end of the feed cable;
- a flexible tubular member comprising a shaft portion and a tip portion, the tip portion located at a distal end of the tubular member and being adapted for insertion into tissue, wherein the applicator and at least part of the feed cable are located within the tubular member; and
- a restriction means arranged to restrict the separation between the applicator and the tip portion of the tubular member,
wherein the restriction means comprises a flexible tethering member extending between the applicator and the tip portion, the tethering member being arranged to restrict at least proximal movement of the applicator relative to the tip portion of the tubular member.

2. The ablation probe of claim 1, wherein the tethering member comprises a flexible filament extending between the applicator and the tip portion of the tubular member.

3. The ablation probe of claim 2, wherein the tethering member is formed from a polymeric material.

4. The ablation probe of any preceding claim, wherein the tethering member is formed from the same material as the tip portion of the tubular member and is embedded within the material of the tip portion.

5. The ablation probe of claim 4, wherein the tip portion and tethering member are reflow bonded together.

6. The ablation probe of any preceding claim, wherein:
the tubular member comprises a tip component coupled to the shaft portion of the tubular member; and
the shaft portion, tip component and tethering member are all formed from the same material.

7. The ablation probe of any preceding claim, wherein the restriction means is arranged to restrict distal movement of the radiating section of the applicator relative to the tip portion of the tubular member.

8. The ablation probe of claim 7, wherein the restriction means comprises an abutment portion of the applicator, the abutment portion arranged to space apart the radiating section of the applicator from the most distal point of the applicator.

9. The ablation probe of claim 8, wherein the abutment portion comprises an anchor point for the tethering member, and optionally wherein the anchor point comprises a hole in the abutment portion arranged to couple with the tethering member.

10. The ablation probe of any proceeding claim, comprising one or more markers comprising a marker material visible using an associated imaging system during use of the ablation probe, wherein the marker material is preferably a material which is radiopaque and/or echogenic, and wherein optionally
a) at least one of the one or more markers is located at a known fixed position relative to the centre of the ablation zone produced by the applicator;
b) at least one of the one or more markers is located at a known fixed position relative to tip; and/or
c) at least one of the one or more markers is located at a position corresponding to a point where the radiation emitted by the applicator is below a threshold safe level.

11. The ablation probe of claim 10, wherein:
a) the tip portion comprises a, or the, marker material and acts as one of the one or more markers, wherein preferably the tip portion comprises a polymeric material doped with the indicator material; and/or
b) the applicator comprises a dielectric resonator comprising a, or the, marker material and acts as one of the one or more markers, wherein preferably the applicator comprises a radiopaque ceramic.

12. The ablation probe of claim 10 or claim 11, wherein the one or more markers include one or more marker components secured to an associated component of the ablation probe, wherein optionally:
a) the one or more marker components comprise a band extending around the feed cable; and/or
b) the one or more marker components comprise a metallic band.

13. The ablation probe of any preceding claim, further comprising a coolant circuit extending along the length of the tubular member and feed cable, wherein the coolant circuit extends through the restriction means to allow passage of coolant around the distal end of the applicator.

14. The ablation probe according to any preceding claim, wherein:
the applicator comprises an antenna section formed integrally with an inner conductor of the feed cable, the antenna section being wrapped around an outer surface of the applicator and mechanically coupled to the applicator by one or more retention features on the outer surface of the applicator.

15. A method of manufacturing a microwave ablation probe, the microwave ablation probe comprising:
- an applicator having a radiating section arranged to emit electromagnetic energy to cause heating in tissue;
- a feed cable having a distal end and a proximal end and being arranged to supply electromagnetic energy to the applicator, the applicator being coupled to the distal end of the feed cable;
- a flexible tubular member, comprising a shaft portion and a tip portion, the tip portion located at a distal end of the tubular member and being adapted for insertion into tissue, wherein the applicator and at least part of the feed cable are located within the tubular member; and
- a restriction means arranged to restrict the separation between the applicator and the tip portion of the tubular member, wherein the restriction means comprises a tethering member extending between the applicator and the tip portion, the tethering member being arranged to restrict proximal movement of the applicator relative to the tip portion of the tubular member,
the method comprising one or both of:
a) bonding the tethering member and the tip portion by reflowing the material of the tethering member and the tip portion; and
b) making the tip portion visible under image guidance by reflowing the material of the tip with a radiopaque material.
